# EUROPEAN PATENT APPLICATION

(11) **EP 2 446 839 A1**
(43) Date of publication of application: **02.05.2012**
(21) Application number: 10774443.5
(22) Date of filing: 17.03.2010
(51) Int. Cl.: A61B 17/122, A61B 17/064

(54) **ENDOVASCULAR SUTURE STAPLE**

(30) Priority: 12.05.2009 BR MU8900911 U
(71) Applicant: Biokyra Pesquisa E Desenvolvimento Ltda, 88036-001 Florianópolis - SC (BR); Lourenço, Marco Antonio, 80250-200 Curitiba - PR (BR)
(72) Inventor: LOURENÇO, Marco Antonio, 80250-200 Curitiba - PR (BR); FACCHINI DE SOUZA, Charles Cristian, 88036-001 Florianópolis - SC (BR); PEREIRA, Fernando Marcelo, 88036-001 Florianópolis - SC (BR); MASIERO FILHO, Isaias, 88036-001 Florianópolis - SC (BR)
(74) Representative: Isern-Jara, Jaime
(86) International application number: PCT/BR2010/000090
(87) International publication number: WO 2010/130016

(57) **Abstract**

"ENDOVASCULAR SUTURE STAPLE", a component utilized for fastening a medical device to the wall of a blood vessel or inside a hollow body organ, may be specifically utilized for fastening vascular grafts on vessel walls, by means of endovascular sutures, comprised of a straight segment on the anterior end or front portion with its end trimmed to a wedge shape or pointed, and the posterior end(12) has a plain, straight, transversal section.

## Description

It is a component used for fastening a medical device on a blood vessel wall or within a hollow body organ. It may be specifically used for fastening vascular grafts on vessel walls, by means of endovascular sutures.

Vascular diseases, such as arterial aneurisms, cause weakening of the vessel wall that may dilate until its rupture. Treatments for this type of diseases utilize vascular or endovascular (endo-prosthesis) grafts made of biocompatible material, such as polyester, ePTFE, stainless steel, Nitinol or gold, among others. Said grafts are fastened to vessels by means of mechanical suture, on traditional techniques, or by means of self-expandable *stents* that exert certain radial force on the internal walls of the vessels, on endovascular techniques.

Hopkinson and Hinchliffe [HINCHLIFFE R, J., HOPKINSON B. R. Current concepts and controversies in endovascular repair of abdominal aortic aneurysms - Journal of Cardiovascular Surgery. # 44, p. 481-502, 2003] describe some types of problems that occur on minimally invasive devices (endo-prosthesis) used in arterial aneurism diseases, highlighting leakages (*endoleaks*) and migration, among them.

Tout III and Tanner (TOUT III H.H., TANNER H.M. A new vascular endostaple: a technical description - Journal of Vascular Surgery, #34, pp. 565-568, 2001) show alternatives for endo-prosthesis fixation in blood vessels The authors describe that desirable characteristics of an endovascular fixation system must comprise; a) a flexible, small insertion catheter; the catheter end must be easily maneuverable, b) the artery wall must be easily penetrated, in despite of the calcification level of aortic wall; c) the staple must resist to displacement during the endo-prosthesis whole life cycle and the intraluminal portion of the staple must have a low profile.

Some patent documents also describe devices for vascular endo-prosthesis fixation. Patent document EP1300121, for example, describes a device created by Parodi, in which the staple shape and placement are similar to those of a screw, within a vessel, by means of an endovascular device.

Patent request document US2004059344, by same inventor (Parodi), describes an improvement on device of previous patent including different types of staples, screws and delivery apparatus. Staples, as well as parts of delivery system, may be manufactured of stainless steel or polymeric material. Parodi highlights that a part of the components responsible for addressing the staples on delivery system of the device is manufactured from a shape memory material. The system described in the patent, allows for placing one staple at a time, and it is necessary a repositioning for placing another staple.

On patent request US2007032863, Bolduc describes a device using helical rigid screw-shaped staples that are fastened with torque movement. Developed staples are shaped in order to allow endo-prosthesis fixation through a *stent* ring. This fixation with endo-prosthesis may be performed by means of a mechanical, magnetic or chemical coupling.

On patent request US2005004582, Edoga and Richard describe a stapler that uses pre-shaped staples manufactured of nitinol or shape memory alloys. Staples have various shapes. Delivery system uses a balloon to press the staples exit against vessel walls. Once placed, the balloon is inflated, and staples are pushed in order to perforate the vessel and the graft walls, fastening the staple to endo-prosthesis. The stapler works with only one staple at a time, and it may be of single use (only one staple) or it may be loaded with several staples. Even loaded with several staples, repositioning is necessary for placing each staple. On inventors' development it is observed a high volume of material on delivery system with big diameters resulting in a high profile.

On patent request US20080262597A1, Xiao and Marilla describe an endovascular prosthesis fixation device that also works with helical fasteners or staples. On the device described by inventors, the endo-prosthesis delivery system has staples carriers that are activated for suturing the endo-prosthesis to the artery. On Xiao's and Merilla's device, staples have not individual controls, it is, all the staples are placed and released simultaneously, making difficult or impossible its effective operation in calcified portions of arteries or in areas with accentuated tortuosities. In one of the inventors' device configurations, the tubes carrying the fixation staples are placed by means of guides connected to endo-prosthesis and to said tubes. According to the inventors, these guides are disconnected or cut after releasing the endo-prosthesis and placing the staples. In another configuration the inventors use a so called tubular limb connected to the tubes carrying the staples. The inventors do not describe construction details about the junction between both parts; they only say, in a general way, that it is a flexible junction.

On the same patent request US20080262597A1, Xiao and Marilla describe the staple or fastener as having a *loop* shape with a pointed end, and the other end is rounded, and it may be manufactured from several materials. In another patent request, US20080262596A1, Xiao details other types of staples or fasteners to be used as endo-prosthesis holders. Xiao describes staples that are similar to those described by Edoga and Richard on the patent request US20050004582A1 and also staples that are similar to those described by Parodi on patent request US20040059344A 1.

On patent request US 006113611A, William J. Amber and Arnold Miller describe a surgical staple and the respective delivery system. The device basically comprises of a guide-tube with a penetrating end, inside of which a helical staple is stored, and it has auxiliary systems for operating the device. In order to place the staple, the guide-tube must perforate the tissue to be sutured and partially release the staple from the other side. Then, the guide-tube is retracted and the staple is completely released. On final configuration, the staple assumes the helical shape, pressing both sides of the tissue.

On patent request W096/9025, Balduc, Krames, Hodges, McCoy and Lunsford show several possible configurations for helical sutures, specifically the spiral-shaped staples. Among the said possibilities, one of them shows a helix-shaped staple with a straight segment at rear portion. This straight segment at its rear portion is shaped in a a manner to facilitate the staple coupling on delivery system.

The endovascular suture staple, reported herein, has an alternative configuration that facilitates its placement, without the penetrating guide-tubes and preventing the staple from penetrating more than once into the tissue. The staple or fastener, reported herein, shows an inventive feature on its construction. It comprises of a spiral spring with defined diameter and number of coils and it has the front end comprised of a straight segment with a sharp point. This straight segment on front portion allows the staple to act as a drilling element, but prevents several perforations to the tissue. The straight segment on the front portion also allows deeper penetration into artery wall when the staple starts its perforation. Moreover, straight segment at staple front portion causes the staple to assume the shape of two parallel helixes, one on each side of the tissue, without the need of using a penetrating guide-tube. The rear end may be plain rectified or it may have a blind, centered orifice for fitting the front end of subsequent staple.

Said endovascular suture staple, reported herein, may be utilized or delivered with the delivery devices described on patent requests by Xiao and Marilla, US200$0262597A1, as well as on patent request submitted to INPI, Brazil, entitled "*Dispositivo de Posicionamento e de Entrega de Componentes Endovasculares*" (Device for endovascular components placement and delivery).

The endovascular suture staple, reported herein, may be manufactured from metallic material, titanium, nitinol, spring steel, stainless steel, or any other material with shape memory and/or superelasticity, as well as from polymeric or copolymeric material. Any selected materials must also be biocompatible.

The straight segment on anterior end or front portion of staple helical body, as a feature of endovascular suture component reported herein, allows a deeper penetration of component during the release procedure and prevents the staple end from doing a second perforation to the tissue, as the case of the staple described on patent request US2008/0262597A1. Thus, the staple assumes the final configuration of helical shape pressing both sides of tissue; configuration similar to that obtained with the system described on patent US006113611A, but without the need of a penetrating guide-tube for its placement, because the said staple, by means of the straight segment on the front portion, perforates the covering and prevents a second perforation.

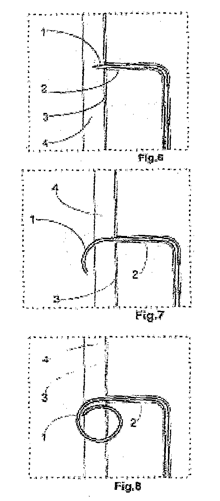

The staple placement may be performed as described on patent request submitted to INPI, Brazil, entitled "*Dispositivo de Posicionamento e de Entrega de Componentes Endovasculares* (Device for endovascular components placement and delivery), showed on figures 6 to 8. The pusher of delivery system is commonly activated until the staple or fastener perforates the vessel and the graft walls. As the staple is pushed by the pusher, the straight segment on the staple front portion prevents the staple from perforating the tissue for the second time. Thus, the staple assumes the shape of helixes parallel to the tissue to be sutured. When two or more coils of the staple penetrate the vessel wall, the delivery system must be retracted; then, the rear portion of the staple is released from delivery system, causing the other coils of staple helical body to remain on inner side of the vessel and the graft. Thus, the staple or fastener will perform a compression effort between the graft and the vessel wall. Note that for placing the staple using the described technique, the guide-tube does not need to perforate the tissue and the graft. The perforation of the tissue and the graft is exclusively performed by the suture staple, and the straight end on anterior portion of the staple causes it to assume the spiral shape in a level parallel to the tissue, it is, the staple does not perform a second perforation of the tissue.

The showed figures facilitate the comprehension about the use of the staple.

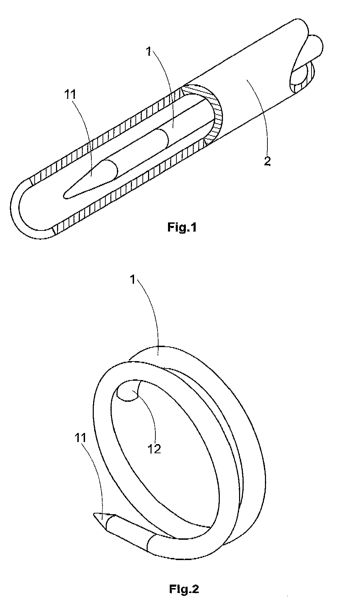

Figure 1 shows a representation of the staple(1) rectified and inserted into the delivery device(2). Due to its superelasticity, it readopts its original helical shape after being expelled from the delivery device(2). It shows the straight segment at the anterior end(11) of the staple(1) helical body, with pointed shape;
Figure 2 shows a representation of the staple(1) with two coils, in its original shape. It shows the straight segment at the anterior end(11) of the staple(1) helical body, with pointed shape and the posterior rend(12);

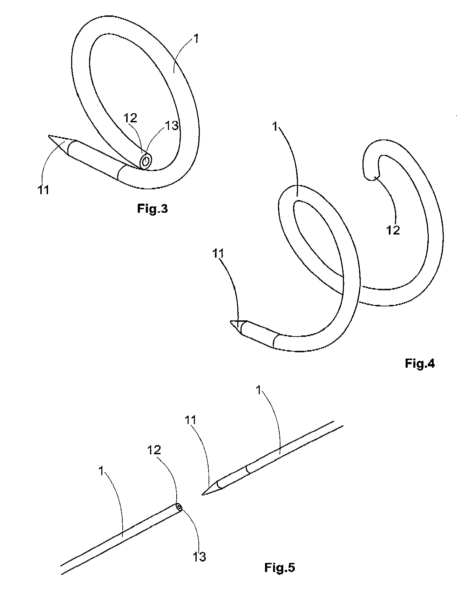

Figure 3 is a representation of staple(1) with only one coil and it shows the posterior end(12) of the staple(1) as a plain, straight section including the blind orifice(13), at central area. It is important to highlight that posterior end(12) may be simply a plain, rectified surface, it is, without orifice.
Figure 4 shows a representation of the staple(1) with two coils, in its definitive shape, with an elongated spiral spring pass.
Figure 5 shows how adjacent staples(1) may be fitted, each other, when they are inside the device(2); it shows the pointed anterior end(11) of a staple(1) fitting in the blind orifice(13) of posterior end(12) of an adjacent staple(1) when the staple has a blind orifice on its posterior end. If the staple does not possess a blind orifice on its posterior end, there will be only a contact between the pointed surface of anterior end(11) of one staple with the plain surface of posterior end of adjacent staple.
Figures 6 and 7 show how the staple(1) acts during the suture of the graft(3) on the vessel(4);
Figure 8 was deleted.

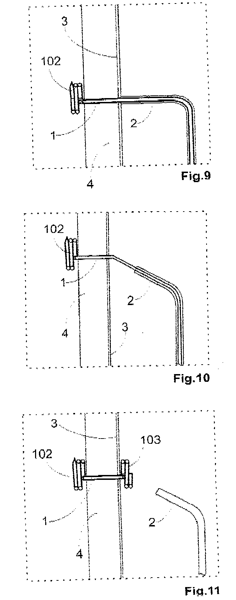

Figure 9 shows, in the section, the coils of anterior end(102) fitted to their definitive position;
Figure 10 shows, in the section, the coils of anterior end(102) fitted to their definitive position and the removal of the delivery device(2) releasing the coils of posterior end(103);
Figure 11 shows, in the section, the coils of posterior end(103) fitted to their definitive position;

The staples(1) are previously shaped into a spiral shape(102 and 103), as a spring, with coil diameters varying from 1.0 to 8.0 mm, the wire diameter varying from 0.1 to 0.7 mm, the pass of the coil varying from 0 to 5 mm and the number of coils varying from 1 to 100. Note that the straight segment at the end of the staple or fastener may vary from 1 to 10 mm. The length of the staples(1) nay vary according to the straight segment, the number of coils and the diameter of coil and wire, depending on the application. Once the lengths are defined and fitted, the staples(1) are rectified; the anterior end(11) of the staple is trimmed to a wedge shape or a pointed shape and the posterior end(12) of said staple(1) has a plain, straight, transversal section, with or without a blind orifice(13) at the center of the section, in order to fit the anterior eztd(11) of the following staple. The orifice may be done by means of special manufacturing techniques. This geometric configuration allows the pusher of the delivery device to push successive staples(1).

Figures 6 and 7 show graphs for the placement of the staple(1) as described before. In this case, the staple(1) is inserted by the movement of the pusher of the delivery system(2), perforating the graft(3) and the wall of the vessel(4) until two or more coils of the anterior end(102) penetrate the vessel(4). Then the delivery device(2) is retracted releasing the coils of posterior end(103), inside the vessel. The coils of the posterior end(103) are in the rear portion of the staple(1). Once the staple(1) is placed, it applies a light compression effort between the wall of the graft(3) and the wall of the vessel(4), contributing to prevent the migration and to increase the sealing of endovascular graft(4).

## Claims

1. "ENDOVASCULAR SUTURE STAPLE", comprising a device, such as a staple(1) or fastener pre-shaped into a spiral spring shape with an anterior end(11) having a sharp point and a posterior end(12)having a plain and straight transversal section; and the said staple(1) is used for performing the suture os a graft(3) in blood vessel walls(4), or for repairing or fastening another device inside a hollow organ of human body, wherein the anterior end(11) of said staple(1) is constituted by a straight segment, with its end trimmed to a wedge shape or pointed;

2. "ENDOVASCULAR SUTURE STAPLE", according to the claim 1 wherein said staple optionally has a blind orifice (13) at the center of the plain, straight, transversal section of the posterior end(12);

3. "ENDOVASCULAR SUTURE STAPLE", according to claim 1, wherein the straight segment on anterior end of staple(1) has a length varying from 1(one) to 10(ten) mm;

4. "ENDOVASCULAR SUTURE STAPLE", according to claim 1, wherein the straight line on anterior end(11) of the staple (1) and in the same place to a wedge shape or pointed;
